# EUROPEAN PATENT SPECIFICATION

(11) **EP 1 838 313 B1**
(45) Date of publication and mention of the grant of the patent: **27.03.2013**
(21) Application number: 05823928.6
(22) Date of filing: 20.12.2005
(51) Int. Cl.: A61K 31/4422, A61K 31/606, A61K 45/06, A61P 1/04, A61P 13/02, A61P 13/08

(54) **PHARMACEUTICAL COMPOSITION COMPRISING NIFEDIPINE AND MESALAZINE FOR THE TREATMENT OF RECTOANAL TENESMUS**
PHARMAZEUTISCHE ZUSAMMENSETZUNG MIT NIFEDIPIN UND MESALAZIN ZUR BEHANDLUNG VON REKTOANALEM TENESMUS
COMPOSITION PHARMACEUTIQUE CONTENANT DE LA NIFEDIPINE ET DE LA MESALAZINE POUR LE TRAITEMENT DU TENESME RECTO-ANAL

(30) Priority: 22.12.2004 IT RM20040631
(43) Date of publication of application: 03.10.2007
(73) Proprietor: Antropoli, Carmine, 81020 Sant'Angelo in Formis, Capua CE (IT)
(72) Inventor: Antropoli, Carmine, 81020 Sant'Angelo in Formis, Capua CE (IT)
(74) Representative: Sarpi, Maurizio
(86) International application number: PCT/IT2005/000748
(87) International publication number: WO 2006/067823

(56) References cited:
- WO-A-98/37886
- ZARAGOZA MARCET A ET AL: "Nephropathy due to mesalazine" REVISTA ESPANOLA DE ENFERMEDADES DIGESTIVAS 2001 SPAIN, vol. 93, no. 8, 2001, page 548, XP008070998 ISSN: 1130-0108
- PAOLUZI P ET AL: "Oral and topical 5-aminosalicylic acid (mesalazine) in inducing and maintaining remission in mild-moderate relapse of ulcerative colitis: One-year randomised multicentre trial." DIGESTIVE AND LIVER DISEASE, vol. 34, no. 11, November 2002 (2002-11), pages 787-793, XP002406067 ISSN: 1590-8658
- DATABASE BIOSIS [Online] BIOSCIENCES INFORMATION SERVICE, PHILADELPHIA, PA, US; 1991, FLEISCHMANN J D ET AL: "CLINICAL AND IMMUNOLOGICAL RESPONSE TO NIFEDIPINE FOR THE TREATMENT OF INTERSTITIAL CYSTITIS" XP002406074 Database accession no. PREV199293021259 & JOURNAL OF UROLOGY, vol. 146, no. 5, 1991, pages 1235-1239, ISSN: 0022-5347

## Description

The present invention relates to a composition including nifedipine and mesalazine in the form of cream, ointmet, gel, suppository, enema and micro-enema that can be used for the treatment of rectoanal tenesmus. As known, tenesmus can appear in association with proctitis, ulcerous rectocolitis, and advanced rectoanal and genitourinary neoplasiae as well as under particular postoperative conditions such as in consequence of low anterior rectum resection syndrome with direct anastomosis or J-reservoir formation of the colic or ileac type (*low anterior resection syndrome) [43].*

In particular, proctitis is an inflammatory process of the mucous membrane of rectum and can extend from the anal margin by a length of about 5-7 cm. Aspecific proctitis represent about 80% of the rectal phlogistic processes and are differentiated from specific proctitis (Ulcerous rectocolitis, Chron's disease, lues, gonococcaemia). Aspecific proctitis are divided in turn according to the possible pathogenic mechanism (diarrhoea, constipation, abuse of evacuative enemas, abuse of topical medicaments (ergotamine), radiotherapy, psychosomatic factors). Among these, also idiopathic proctitis where the pathogenic moment cannot be traced back to other possible origins should be considered. A clinical sign common to these patients is tenesmus.

In particular, tenesmus can be a very frequent, bothersome disorder in patients with ulcerous rectocolitis which is still not in acute phase and under the control of an usual therapy.

Ulcerous rectocolitis firstly concerns rectum with the greatest frequency. A progressive extension of the inflammation to left colon (proctosigmoiditis) and in worst cases the whole colon (pancolitis) can occur in a lower proportion of patients until the appearance of very serious colic, systemic complications which can require to go into surgery with the danger of death for the patient. Nifedipine [dimethyl ester of 1,4-dihydro-2,6-dimethyl-4-(2-nitrophenyl)-3,5-piridino carboxyl acid] is a well-known active principle used in the treatment of ischemic cardiopathy and arterial hypertension. It has been administered via mouth for years in the treatment of hypertension and cardiovascular pathologies as it induces a relaxation of the vascular paries.

The interaction of nifedipine with biological systems is mostly due to the fact that it acts as calcium antagonist like dihydropiridine. Calcium antagonist drugs represent a heterogeneous group of molecules which share the property of blocking the selective channels for the calcium ion of L type which are voltage-depending at the cellular membranes [3, 4, 19, 20]. As the flows of calcium ion from the above-mentioned channels are indispensable for the regulation of the levels of intracellular calcium ion which acts as intracellular activator messenger, and for the regulation of the usually negative cell membrane potential, the calcium antagonists act as inhibitors of some cell functions and the exciting process through the cell membrane [3, 19]. The calcium antagonists reduce the contractility of the cardiac muscle and release the unstriated muscle of the vascular paries in vivo.

The effectiveness of nifedipine can be ascribed to such an action mechanism under morbid conditions of the anal canal associated to hypertonus of the inside anal sphincter such as anal fissure and generally rectalgia.

The myorelaxing action of nifedipine to unstriated muscle of the vas paries has also been proved to unstriated rectoanal muscle, particularly the inside anal sphincter. Cook and Coll studied the effects of nifedipine on 24 preparations of anal unstriated and circular rectal sphincter muscle in a pig to evaluate the effects of modulating the tone and the contractions induced by the antagonist noradrenaline and carbachol. They reported a significant reduction in the tone of the anal sphincter muscle fibres and a reduction in contractility induced by the antagonists of the fibres of the circular rectal unstriated muscle [17]. The same study has been repeated and confirmed for humans on preparations of unstriated muscle from 10 patients subjected to surgical operation [18].

It has been shown that morbid conditions such as anal fissure and haemorrhoids are united by a hypertonus of the inside anal sphincter [5].

Moreover, the anal sphincter hypertonus of the above-mentioned morbid conditions, such as rectalgia and tenesmus associated to proctitis, anal fissure, haemorrhoids, is associated to other pre-existing causes such as a physical injury (hard stools), anodermous lacerations (in case of acute enteritis), chronic hypertonus of the secondary internal anal sphincter with ulceration in intra-anal thrombosis, cryptitis and abuse of laxatives [6]. Owing to this, the formation of an inflammatory infiltrate from a rectoanal injury involves a continuous hypertonus of the internal anal sphincter, while cramp contractions are the response of the external anal sphincter. A vicious circle is set up with a poor tendency to recovery.

Therefore, the recovery from hypertonus on a pharmacological basis allows surgical operations with possible appendant complications to be avoided as well as proctologic pathologies on a functional basis [6] and a number of disorders due to organic pathologies such as rectal tenesmus upon ulcerous rectocolitis or neoplasia [7, 8, 9] to be treated.

Nifedipine has not only a valid myorelaxing effect on the unstriated muscle [3, 18, 19, 25] but experimental tests have also detected that nifedipine and some calcium antagonists show an anti-inflammatory action [21, 22, 26] and the capability of modulating the microcirculatory system, and can act topically [27] as well.

These experimental tests that have detected the anti-inflammatory action [21] and the capability of modulating the microcirculatory system [23] of nifedipine suggest that the effects of a topical application of nifedipine act as: anti-inflammatory and induced immunoregulators with a 1% concentration in the Langerans cells of the rat skin [21]; regulators of the microcirculatory system in the hamster's model [23] and the microcirculatory system of the cortical pia of the cat subjected to focal ischemic injury [34]; vasodilators of the arteriolae of the microcirculatory system of rat mesentery [35] which is effective by topical application in the prevention of the microvascular spasm in the isolated, epigastric flap of rat [36]. These and other comments can help to act on the inflammatory component supporting the vicious circle which is responsible for the pathogenic mechanism of the proctologic pathologies supported by hypertonus of the internal anal sphincter.

Furthermore, it is known from experimental tests [37, 38] that the costive action of nifedipine induced by the regulation of the wandering intestinal myoelectrical complexes could be a further benefit for the control of the diarrhoeic symptomatology of the ulcerous rectocolitis in proctitis and ischemic colitis.

Nifedipine is usually administered both for oral use [10, 11] and topical application of a cream in rectoanal disorders WO 98/37886 and pathologies such as haemorrhoidal thrombosis [12, 13] and anal fissure [14, 15, 16].

It is known that nifedipine is able to provide a myorelaxing effect of the rectoanal unstriated muscle after oral administration, but the topical application is preferable as the oral administration can reduce its specificity because the drug interacts concurrently with internal anal sphincter and rectal, longitudinal, unstriated muscle [17, 18]. Therefore, the topical administration of nifedipine near the anal sphincter muscle is able to reduce the tone and the contractions of the internal anal sphincter by a greater specificity through the inhibition of the Ca2+ flow into the sarcoplasm of the unstriated musculature and the pressures detectable in the anal canal [14, 17, 18].

As well as being presently accepted for the clinical use in the treatment of the anal fissure [33], the therapeutic topical action of nifedipine was also observed through rhinal [27], dermal and plaster applications, and documented by in vitro [28, 29], in vivo [32] studies and researches on animals [30,31]. Nifedipine is capable of being administered by topical application in association with other active principles consisting of local anaesthetics such as lidocaine, carbocaine and the like.

In fact, the same Applicant has obtained the registration of the preparation Antrolin^{®}, a rectal cream containing 0.3% nifedipine and 1.5% lidocaine, (trademark filed in Italy with the Ministry of Health), with indications for the treatment of the anal fissure and rectalgia associated to sphincter hypertonus according to WO 98/37886.

Mesalazine (5-aminosalicylic acid, 5-ASA) is presently the drug of reference that exhibits its utility in holding the clinical remission in light, moderate, ulcerous rectocolitis with localization at the rectum and left colon. Such active principle exhibits a topical anti-inflammatory effect on the injured tracts of the intestinal mucous membrane. Its presence in the intestinal lumen at enough concentration inhibits effectively the biosynthesis of the arachidonic-acid metabolism derivatives such as prostaglandin E2, thromboxane B2 and leucotrienes, the levels of which are considerably high in biopsy samples taken from rectal mucous membrane of patients with a new acute phase of ulcerous colitis.

Mesalazine is the active fraction of salicylazosulfopiridine (SASP), a drug widely used in these clinical forms in the past but now of limited use because of the frequent unfavourable events. Mesalazine is in the traditional pharmaceutical form of enema in doses of 2 g in 50 ml and 4 g in 100 ml as ready-to-use enema, or in doses of 2 g mesalazine for extemporary preparations of a 50 ml enema. Foam unit preparations of 5-ASA in doses of 2 g or 4 g in 10 or 20 ml foam excipient, and suppository preparations of 1 g consisting of 500 mg 5-ASA and 500 mg 5-ASA gel monodose have been recently put on the market to provide an immediate anti-inflammatory effect in case of ulcerous injuries of the pis distal colon tracts and anus and rectum as well. The absorption of Mesalazine by the colon is low. The substance is mostly evacuated with stools and the plasmatic levels are low after oral administration. The mesalazine administered to humans via rectum by enema or suppository has a very poor systemic absorption of about 10% of the administered dose in subjects with acute enteral inflammatory forms and acts essentially topically. Enemas and suppositories are preparations that allow mesalazine (5-aminosalicylic acid, 5-ASA) to be administered via rectum, thus ensuring a prompt anti-inflammatory effect in ulcerous injuries of the intestine end portion as well as a particularly high local and general tolerance.

The content of DL-50 of mesalazine, after oral administration to rat, is 4594 mg/kg. The local, systemic tolerability of the enema in the rectum-sigmoid mucous membrane of the animal is good at any dose (until 6g/kg). Diarrhoeic phenomena depending on the volume, however, appear immediately after the administration of the higher doses. The local tolerability of suppositories via rectum proved also good. In addition, mesalazine has no mutagenic affinity.

Furthermore, the pharmaceutical-economic analysis confirmed that the use of mesalazine for local use helps to reduce the treatment cost [1, 2].

However long-term steroid therapy (47, 48) and low efficacy of long term mesalazine therapy in control of pain and tenesmus may cause problems (49, 50, 51, 52). In the light of the known state of art, it is self-evident that the treatment of the anal, rectal tenesmus is a particularly important problem of difficult solution. In particular, the today's efforts are directed to the set up of even more efficacious pharmaceutical compositions to avoid side effects of long term mesalazine therapy and the surgical operation of pathologies associated to anal tenesmus, as already described.

A further, particularly pressing requirement is to simplify the therapeutic protocols and to reduce the number of drugs to be administered to the patient for the treatment of the same pathology.

A further highlighted problem is to produce new administering forms capable of further increasing locally the effectiveness of the active principles, thus reducing the parenteral administration and the side effects thereof.

The technical problems summarized above have been solved by producing a composition characterized by the association of two known active principles in only one proprietary medicine for the treatment of the anal tenesmus, the specific rectal tenesmus associated to ulcerous colitis of the type L-UC and UP as well as the treatment of the aspecific rectal tenesmus associated to aspecific proctitis, advanced rectal and genitourinary (vesical, prostatic, utero-ovarian) neoplasiae.

Therefore, the object of the present invention is a pharmaceutical composition including nifedipine and mesalazine and conventional, pharmaceutical acceptable excipients in the form of cream, ointment, gel, suppository, enema and micro-enema.

It has been experimentally shown up that the association of the two active principles in said compositions is typically synergistic. In fact, mesalazine holds its effectiveness as antiphlogistic [1, 2], and nifedipine carries out its activity of calcium antagonist at several levels: inhibition of the Ca++ flow through the cell membranes which causes the reduction of the mechanical contraction of the muscular fibres in the sarcoplasm of the muscular unstriated cell, an anti-inflammatory, immuno-regulating activity and a modulating activity regulating the microcirculatory system regarding the capillary permeability.

Owing to the combination with nifedipine, the activity of 5-ASA is strengthened not only because of an additional effect due to the greater vasodilatation induced locally by nifedipine as well as the increased haematic flux and the oxygen perfusion into seriously inflamed tissues being a prey to ulceration processes, cryptic onset of abscess, and necrosis that help the cell regeneration and healing processes. Additionally and not in the second place, the presence of nifedipine allows lower concentration of 5-ASA to be administered.

The presence of two active principles allows the therapy to be simplified. In this case, in fact, the patient receives daily or twice a day only one preparation including nifedipine and 5-ASA unlike the traditional protocols according to which he/she is bound to undergo at least four times a day a therapy with nifedipine cream to relieve anal tenesmus and hypertonus and additional microenemas of 5-ASA to fight against bleeding and rectalgia.

Clinical tests concerning the separate administration of the two drugs for oral use did not detect any pharmacological interaction.

There is one case report in Literature (Zaragoza Marcet et Al: "Nephropathy due to mesalazine " REV ESP ENFERM DIG 2001; 93, (8): 548), in which the concomitant use of nifedipine and mesalazine is described in a patient with three chronic diseases (Crohn's diseases, ischemic cardiopathy and spondilitis). The patient used nifedipine and mesalazine chronically and only for oral way. This patient, at the later stage, interrupted the use of mesalazine, and not of nifedipine, for renal injury. On the basis of the above document, wherein the use of the two compounds is disclosed, without any indication as quantity and as period of administration, the skilled person would deem that the association between the two compounds lead to a non-beneficial effect. On the contrary, the present application describes an association between two well-known substances nifedipine and mesalazine in form of cream, ointment, gel suppository, enema and micro-enema for a local, not oral, treatment, that is for endorectal administration."

Furthermore the local separated application of the two active principles is of safe use with dosages and concentrations proposed for clinical use [39].

Three important researches were made to set up the composition of the invention.

The first research studied the activity of the active principle, namely the nifedipine cream applied locally by microenemas of 3 g containing two different concentrations, i.e. 0.3% and 0.2%, to treat aspecific, idiopathic proctitis of a limited number of patients. The concentrations were selected on the base of previous works using nifedipine locally on patients suffering from haemorrhoids and anal fissure.

A limited group of 10 patients suffering from tenesmus and burning feeling during or after defecation, with or without rectal haemorrhage, were subjected to andoscopic, bioptic analysis resulting in a diagnose of aspecific, idiopathic proctitis with a marked hyperaemia of the rectal mucous membrane associated or not to the presence of areas of disepithelization, ulceration or friableness. Five patients were given 0.3% nifedipine cream and further five patients 0,2% nifedipine cream by microenemas of 3 g administered twice a day for 14 days. The 10 patients (five men and five women) were 36.8 ± 9.3 years old on average. After a few days the remission of the symptomatology in the group of 0.2% nifedipine was precocious and after 14 days it was effective in four (80%) of the five patients. The remission of the symptomatology was reached in the fifth patient after a therapy for 7 additional days. The remission of the symptomatology in the group of 0.3% nifedipine was also precocious and effective in all five treated patients after 14 days. The endoscopic, histological normalization (normal complexion of the mucous membrane, no ulcer, friableness and bleeding, minimum lympho-plasmocyte infiltration, minimum erosion phenomena) was reached in 3 of 5 patients treated by 0.2% nifedipine and in 4 of 5 patients treated by 0.3% nifedipine. No side systemic effects or significant rectal haemorrhage were found in the totality of 10 patients treated by nifedipine.

The second therapy research studied the activity of the association of 9 mg (0.3%) nifedipine and 2 g mesalazine in a cream administered by 3 g microenema twice a day for 14 days. 10 patients were studied suffering from ulcerous rectocolitis localized at the rectum and detected by endoscopy and histology under quiescence but with a clinical condition of tenesmus not responsive to mesalazine alone or steroids by local action. The remission of the symptomatology in this limited group of 10 patients was precocious and in eight (80%) of ten patients effective after 14 days. The remission of the symptomatology in remaining 2 patients was reached after a therapy for 7 additional days. No side systemic effects or significant rectal haemorrhage were found in the totality of 10 patients treated by associated nifedipine and mesalazine.

Finally in the third example, (Antropoli C. et al. Preliminary efficacy and tolerability assessment of the association mesalazine 1 g plus nifedipine 10 mg in the treatment of tenesmus in patients with distal ulcerative colitis. In : Proceedings 16th UEGW 2008 Austria:Vienna 18-22 October 2008. Abstract submitted for publication.) the applicant was the main investigator of an open trial involving two Italian and two Ukraine centers. The primary objective of this study was to assess the clinical efficacy of the association of mesalazine 1 g plus nifedipine 10 mg in 50 ml micro-enemas in patients affected by distal ulcerative colitis with tenesmus and ≥4 non-productive bowel movements, treated with stable doses for at least one month of mesalazine alone or background therapies, if any. Complete regression of tenesmus was assessed as the decrease in non-productive bowel movements by at least 50% in a stable manner at day 5. The secondary efficacy variable was the evaluation of the tolerability and effect on the patients' quality of life as assessed at final visit compared to baseline by the statement of the enema retention improvement (retention time) and the "urgency" improvement (100 mm VAS - ≥50% reduction in VAS score). 30 patients of both sexes, aged ≥18 years were enrolled (20 males, 48.23 years mean age ± 15.52 S.D.). Mesalazine 1 g and nifedipine 10 mg in 50 ml micro-enemas were administered twice daily by the endo-rectal route for 15 days. Stable reduction in non-productive bowel movements ≥50% at Day 5 was achieved by 15 (50%) patients. Mean enema retention time increased compared to baseline, from 0.76 to 2.65 hours at day 15 with statistical significance (p < 0.0005). 21 (70.0%) patients were responders at Day 15 in "urgency" symptoms with high statistically significant trend (p < 0.0001) towards reduction of VAS score. In particular, a statistical significant improvement was observed at Day 5 (mean score, 5.25 ± 2.09 SD; p<0.0001) and 15 (mean score, 1.78 ± 2.16 SD; p < 0.0001), compared to baseline (mean score, 7.18 ±1.92 SD). Evaluating the number of productive and non-productive bowel movements, respectively 12 (40%) and 25 (83,3%) patients were responders at Day 15 with statistical significant trend (p <0,0001) for the reduction of the non productive movements. No serious adverse events or relevant laboratory parameters changes occurred during the study.

Despite the statistical limits of the number of patients, according to three said examples (nifedipine alone and combination of nifedipine and mesalazine in two studies), the clinical control is obtained for about 80% of the patients in two weeks and for 100% in three weeks in comparison with 55/64% obtained by mesalazine alone in 4-8 weeks, as indicated in a leading study. [Paoluzi P et al: "Oral and topical 5-aminosalicylic acid (mesalazine) in inducing and maintaining remission in mild-moderate relapse of ulcerative colitis: One-year randomised multicentre trial." Digestive and Liver Disease, vo.34, no.11, November 2002 (2002-11), pages 787-793)]. The results of these preliminary studies on a new association of mesalazine plus nifedipine may support the efficacy and safety of the study product in management of rectoanal tenesmus and symptoms associated to distal ulcerative colitis, manifestly due to a synergism between nifedipine, a calcium antagonist, and anti-inflammatory activity of mesalazine.

The clinical studies so far disclosed have brought to the development of a pharmaceutical composition comprising nifedipine in a gram concentration between 6x10⁻³ and 0.3 and mesalazine in a gram concentration between 0.1 and 30.

The pharmaceutical composition is added with suitable pharmacologically acceptable excipients and packed preferably in the form of cream, ointment, gel, suppository, enema, and micro-enema, where excipients.

### BIBLIOGRAPHY

[1] Cohen RD, Woseth DM, Thisted RA, Hanauer SB. A meta-analysis and overview of the literature on treatment options for left-sided ulcerative colitis and ulcerative proctitis. Am. J. Gastroenterol. 2000 May; 95[5]:1263-76.
[2] Lim WC, Hanauer SB. Controversies with aminosalicylates in inflammatory bowel disease. Rev. Gastroenterol. Disord. 2004 Summer; 4(3)]:104-17.
[3] Katz AM, Hager WD, Messineo FC, Pappano AJ. Cellular actions and pharmacology of the calcium channel blocking drugs. Am. J. Med. 1984 Aug 31; 77(2B):2-10.
[4] Triggle DJ. Calcium, calcium channels, and calcium channel antagonists. Can. J. Physiol. Pharmacol. 1990 Nov; 68(11):1474-1481.
[5] Arabi Y, Alexander-Williams J, Keighley MR. Anal pressures in haemorrhoids and anal fissure. Am. J. Surg. 1977 Nov; 134(5):608-610.
[6] Klug W, Knoch HG. Comparing dilator efficacy in the treatment of acute anal fissures. Colo-proctology 1993; 1:22-28.
[7] McLoughlin R, Using Nifedipine to treat tenesmus. Palliative Med. 1997; 11:419-421.
[8] Loder PB, Kamm MA, Nicholls RJ, Phillips RK Reversible chemical sphincterotomy by local application of glyceryl trinitrate. Br. J. Surg. 1994 Sep; 81(9):1386-1389.
[9] Keighley MR, Buchmann P, Minervini S, Arabi Y, Alexander-Williams J Prospective trials of minor surgical procedures and high-fibre diet for haemorrhoids. Br. Med. J. 1979 Oct 20; 2(6196):967-969.
[10] Chrysos E, Xynos E, Tzovaras G, Zoras OJ, Tsiaoussis J, Vassilakis SJ. Effect of nifedipine on rectoanal motility. Dis. Colon Rectum 1996 Feb; 39 (2) :212-216.
[11] Cook TA, Humphreys MM, Mortensen NJ. Oral nifedipine reduces resting anal pressure and heals chronic anal fissure. Br. J. Surg. 1999 Oct; 86(10):1269-73.
[12] Perrotti P, Antropoli C, Noschese G et al. Topical nifedipine for conservative treatment of acute haemorrhoidal thrombosis. Colorectal Disease 2000; 2:18-21.
[13] Perrotti P, Antropoli C, Molino D, De Stefano G, Antropoli M. Conservative treatment of acute thrombosed external haemorrhoids with topical nifedipine. Dis. Colon Rectum 2001 Mar; 44(3):405-9.
[14] Antropoli C, Perrotti P, Rubino M, Martino A, De Stefano G, Migliore G, Antropoli M, Piazza P. Nifedipine for local use in conservative treatment of anal fissures: preliminary results of a multicenter study. Dis. Colon Rectum 1999 Aug; 42(8):1011-5.
[15] Perrotti P, Bove A, Antropoli C, Molino D, Antropoli M, Balzano A, De Stefano G. Attena F. Topical nifedipine with lidocaine ointment vs. active control for treatment of chronic anal fissure: results of a prospective, randomized, double-blind study. Dis. Colon Rectum 2002; 45:1468-1475.
[16] Ezri T, Susmallian S, Topical Nifedipine vs. Topical Glyceryl Trinitrate for Treatment of Chronic Anal Fissure. Dis. Colon Rectum 2003; 46(6):805-808 2003; 46(6):805-808.
[17] Cook TA, Brading AF, Mortensen NJ Differences in contractile properties of anorectal smooth muscle and the effects of calcium channel blockade. Br. J. Surg. 1999; 86:70-5.
[18] Cook TA, Brading AF, Mortensen NJ Effects of nifedipine on anorectal smooth muscle in vitro. Dis. Colon Rectum 1999; 42:782-7.
[19] Opie LH. Calcium channel antagonists in the treatment of coronary artery disease: fundamental pharmacological properties relevant to clinical use. Prog. Cardiovasc. Dis. 1996 Jan; 38(4):273-290.
[20] Taira N. Differences in cardiovascular profile among calcium antagonists. Am. J. Cardiol. 1987 Jan 30; 59[3]:24B-29B.
[21] Katoh N, Hirano S, Kishimoto S, Yasuno H. Calcium channel blockers suppress the contact hypersensitivity reaction [CHR] by inhibiting antigen transport and presentation by epidermal Langerhans cells in mice. Clin. Exp. Immunol. 1997; 108: 302-308.
[22] De Vries GW. The antiinflammatory activity of topically applied novel calcium-channel antagonists. Inflammation 1995:19(2), 261-275.
[23] Oshiro H. L-type calcium channel blockers modulate the microvascular hyperpermeability induced by platelet-activating factor in vivo. J. Vasc. Surg. 1995; 22: 732-739.
[24] Kvietys PR, Granger DN. Effect of volatile fatty acids on blood flow and oxygen uptake by the dog colon. Gastroenterology. 1981 May; 80(5 pt 1):962-9.
[25] Plante P. Bladder contraction. Physiopathology, therapeutic effects. I. The bladder muscle. Structure. Electrical properties. Intrinsic regulation] J. Urol. (Paris). 1983; 89(5):287-94.
[26] Fleischmann JD. Clinical and immunological response to nifedipine for the treatment of interstitial cystitis. J. Urol. 1991; 146:1235-1239.
[27] Landau AJ. Intranasal delivery of cardiovascular agents: an innovative approach to cardiovascular pharmacotherapy. Am Heart J. 1994; 127(6), 1594-1599
[28] Ruan LP, Zheng JM. Research on nifedipine patch. Yao Hsueh Hsueh Pao 1991; 26:286-92.
[29] Kobayashi D, Matsuzawa T, Sugibayashi K, Morimoto Y, Kobayashi M, Kimura M. Feasibility of use of several cardiovascular agents in transdermal therapeutic systems with 1-menthol-ethanol system on hairless rat and human skin. Biol. Pharm. Bull. 1993; 16:254-8.
[30] McDaid DM, Deasy PB. An investigation into the transdermal delivery of nifedipine. Pharm. Acta Helv. 1996; 71:253-8.
[31] Kondo S, Yamanaka C, Sugimoto I. Enhancement of transdermal delivery by superfluous thermodynamic potential. III. Percutaneous absorption of nifedipine in rats. J. Pharmacobiodyn. 1987; 10:743-9.
[32] Eldridge JE, Burdick DC, Jones RH, Hossack KF. Comparison of nitroglicerine patches and nifedipine. J. Cardiovasc. Pharmacol. 1987; 10:315-319.
[33] Madoff RD, Fleshmann JW. American Gastroenterological Association. Technical review on the diagnosis and care of patients with anal fissure. Gastroenterology 2003; 124:235-245.
[34] Brandt L. Effects of topical application of a calcium antagonist (nifedipine) on feline cortical pia microvasculature under normal conditions and in focal ischemia. J. Cereb. Blood Flow Metab. 1983; 3:44-50.
[35] Araki H, Itoh M, Nishi K. Effects of nipradilol on the microvascular tone of rat mesentery: comparison with other beta-blockers and vasodilators. Arch. Int. Pharmacodyn. Ther. 1992; 318:47-54.
[36] Weinzweig N, Lukash F, Weinzweig J. Topical and systemic calcium channel blockers in the prevention and treatment of microvascular spasm in a rat epigastric island skin flap model. Ann. Plast. Surg. 1999; 42:320-6.
[37] Yousif F, Triggle DJ. Functional interactions between organic calcium channel antagonists in smooth muscle. Can. J. Physiol. Pharmacol. 1985; 63:193-195.
[38] Thollander M, Hellstrom PM, Svensson TH Dihydropyridine calcium channel antagonists disrupt migrating myoelectric complexes and counteract intestinal disorders associated with morphine withdrawal diarrhea. Scand. J. Gastroenterol. 1993 Feb; 28(2):137-144.
[39] Mahdi G, Israel DM, Hassall E. Cyclosporine and 6-mercaptopurine for active, refractory Crohn's colitis in children. Am. J. Gastroenterol. 1996 Jul; 91(7):1355-9.
[40] Echizen H, Eichelbaum M. Clinical pharmacokinetics of verapamil, nifedipine and diltiazem. Clin. Pharmacokinet. 1986; 11:425-449.
[41] Namiki N, Yokoyama H, Moriya K, Studies on improvement of pharmaceutical preparations prescribed in Hospitals. V. Nifedipine hollow type suppository. Drug Des. Deliv. 1986 Nov; 1(2):167-73.
[42] Kleinbloesem CH, van Harten J, de Leede LG, van Brummelen P, Breimer DD. Nifedipine kinetics and dynamics during rectal infusion to steady state with an osmotic system. Clin. Pharmacol. Ther. 1984 Sept; 36(3):396-401.
[43] van Duijvendijk PA, Slors F, Taat CW, et al. prospective evaluation of anorectal function after total mesorectal excision in patients with a rectal carcinoma. Surgery 2003; 133:56-65.
[44] Angelico P, Guarneri L, Fredella' B, Testa R. In vivo effects of different antispasmodic drugs on the rat bladder contractions induced by topically applied KCl. J. Pharmacol. Toxicol. Methods 1992; 27:33-39.
[45] Zar MA, Iravani MM, Luheshi GN. Effect of nifedipine on the contractile responses of the isolated rat bladder. J. Urol. 1990; 143:835-839.
[46] Patz J, Jacobsohn WZ, Gottschalk-Sabag S, Zeides S, Braverman DZ. Treatment of refractory distal ulcerative colitis with short chain fatty acid enemas. Am. J. Gastroenterol. 1996 Apr; 91(4):731-4.
47) Hanauer SB, Stathopulos O. Risk-benefit assessment of drugs used in the treatment of inflammatory bowel disease. Drug Safety 1991; 6; 192-219.
48) Bansky O, Buhler H, Stamm B et al. Treatment of distal ulcerative colitis with beclomethasone enemas: high therapeutic efficacy without endocrine side effects. Dis Colon Rectum 1987; 30:288.
49) Lim WC, Hanauer SB, Controversies with aminosalycilates in inflammatory bowel disease. Rev Gastroenterol Disord 2004; 5:104-117.
50) harting JW. New developments in the pharmacotherapy of inflammatory bowel disease. Pharm Weekbl 1992; 14: 275-86.
51) Ardizzone S, Doldo P., Ranzi T et al. Mesalazine foam (Salofalk foam) in the treatment of active distal ulcerative colitis. A comparative trial vs Salofalk enema. The SAF-3 study group. Ital J Gastroenterol Hepatolo. 1999; 31: 677-84.
52) Baum CA, Biddle WL, Miner PB Jr. Faliure of 5-aminosalicylic acid enemas to improve chronic radiation proctitis. Dig Dis Sci. 1989 May; 34 (5): 758-60.

## Claims

1. A pharmaceutical composition including nifedipine, mesalazine, and suitable pharmaceutically acceptable excipients, in a concentration able to perform the desired pharmacological action, in the form of cream, ointment, gel, suppository, enema, and micro-enema.

2. The pharmaceutical composition according to claim 1, **characterized in that** nifedipine is present in a gram concentration between 6x10⁻³ and 0.3, and mesalazine between 0.1 and 30.

3. The pharmaceutical composition according to the preceding claims, **characterized in that** the composition includes additionally at least one compound selected from the group comprising lidocaine, carbocaine, non-steroidal anti-inflammatory, and short-chain fat acids (SCFA).

4. Use of the pharmaceutical composition of claims 1 to 3 for the preparation of a medicine for the treatment of rectoanal tenesmus.

5. Use of the composition according to claim 1 for the preparation of a medicine for the treatment of specific and aspecific rectoanal tenesmus.

## Patentansprüche

1. Pharmazeutische Zusammensetzung, enthaltend Nifedipin, Mesalazin und geeignete pharmazeutische verträgliche Hilfsstoffe, in einer Konzentration, um die gewünschte pharmakologische Wirkung zu erhalten, in Form einer Creme, einer Salbe, einer Gels, eines Zäpfchens, eines Klistiers und eines Mikro-Klistiers.

2. Pharmazeutische Zusammensetzung nach Anspruch 1, **dadurch gekennzeichnet, dass** Nifedipin in einer Grammkonzentration von 6x10⁻³ und 0,3 und Mesalazin in einer Grammkonzentration von 0,1 und 30 vorliegt.

3. Pharmazeutische Zusammensetzung nach einem der vorhergehenden Ansprüche, **dadurch gekennzeichnet, dass** die Zusammensetzung zusätzlich wenigstens eine Verbindung enthält, ausgewählt aus der Gruppe, umfassend Lidocain, Carbocain, nichtsteroidale anti-inflammatorische und kurzkettige Fettsäuren (SCFA).

4. Verwendung der pharmazeutischen Zusammensetzung nach einem der Ansprüchen 1 bis 3 zur Herstellung eines Arzneimittels zur Behandlung des rektoanalen Tenesmus.

5. Verwendung der Zusammensetzung nach Anspruch 1 zur Herstellung eines Arzneimittels zur Behandlung des spezifischen und aspezifischen rektoanalen Tenesmus.

## Revendications

1. Composition pharmaceutique comprenant de la nifédipine, de la mésalazine et des excipients pharmaceutiquement acceptables, dans une concentration capable d'effectuer l'action pharmacologique désirée, sous la forme de crème, onguent, gel, suppositoire, lavement et micro-lavement.

2. Composition pharmaceutique selon la revendication 1, **caractérisée en ce que** la nifédipine est présente dans une concentration en gramme entre 6x10⁻³ et 0,3, et la mésalazine entre 0,1 et 30.

3. Composition pharmaceutique selon les revendications précédentes, **caractérisée en ce que** la composition comprend de plus au moins un composé sélectionné dans le groupe comprenant lidocaïne, carbocaïne, anti-inflammatoire non stéroïdien et acides gras à courte chaîne (SFCA).

4. Utilisation de la composition pharmaceutique selon les revendications 1 à 3 pour la préparation d'un médicament pour le traitement du ténesme recto-anal.

5. Utilisation de la composition selon la revendication 1 pour la préparation d'un médicament pour le traitement du ténesme recto-anal spécifique et aspécifique.
